# EUROPEAN PATENT SPECIFICATION

(11) **EP 4 676 363 B1**
(45) Date of publication and mention of the grant of the patent: **18.11.2026**
(21) Application number: 24717858.5
(22) Date of filing: 06.03.2024
(51) Int. Cl.: A61B 17/80

(54) **PECTUS BAR AND STABILIZER DEVICES**
BRUSTSTANGE UND STABILISIERUNGSVORRICHTUNGEN
BARRE DE PECTUS ET DISPOSITIFS STABILISATEURS

(30) Priority: 06.03.2023 US 202363450202 P
(43) Date of publication of application: 14.01.2026
(73) Proprietor: Zimmer Biomet CMF And Thoracic, LLC, Jacksonville, Florida 33218 (US)
(72) Inventor: TESTON, Kevin Lee, Jacksonville, Florida 32218 (US); GARCIA, Saddy Rodolfo, St. Augustine, Florida 32084 (US); DAVIS, Barclay R., Jacksonville, Florida 33218 (US); WILCOX, Bryan, St. Augustine, Florida 32092 (US); SOROKA, Nicholas, Jacksonville, Florida 32246 (US); KNOWLES, Ely Lucus, Jacksonville, Florida 33218 (US); O BRIEN II, T. Shay, St. Johns, Florida 32259 (US)
(74) Representative: Taor, Simon Edward William
(86) International application number: PCT/US2024/018732
(87) International publication number: WO 2024/186931

(56) References cited:
- US-A1- 2017 156 759
- US-A1- 2018 021 074
- US-A1- 2021 022 782

## Description

### CLAIM OF PRIORITY

This patent application claims the benefit of priority, under 35 U.S.C. Section 119(e), to first named inventor, Shay O'Brien, U.S. Patent Application Serial Number 63/450,202, entitled "PECTUS BAR AND STABILIZER DEVICES AND METHODS," filed on March 6, 2023.

### BACKGROUND

Pectus excavatum and pectus carinatum are conditions affecting a human rib cage which can result from a congenital disorder or injury. In some cases of pectus excavatum and pectus carinatum, physicians install hardware into a patient's ribcage to reshape the ribcage. During a procedure a pectus excavatum correction procedure, one or more pectus bars can be inserted into a thoracic cavity of the patient, at least partially intrathoracically. The bar can then be flipped to force the sternum and rib cage into a proper position, and ends of the bar(s) can be secured to one or more ribs. US2021022782 discloses a flail chest stabilization device is provided that includes a pair of lateral bridges, a pair of main stabilization bars, a screw and slider-element connector assembly, and a slider introducer tool, where the slider introducer tool positions the slider-element, where the main stabilization bars are connected by the screw and slider connector assembly to the lateral bridges, where the stabilization bars are in a parallel configuration or crossed configuration. US2018021074 discloses an apparatus for the correction of chest deformities is provided and includes a rib implant for mounting to a rib, a sternal implant for mounting to a sternum, and a first elongate member. The rib implant includes a first aperture having a first axis. The sternal implant includes a second aperture having a second axis. The first elongate member has a first end adjustably received in the first aperture and a second end adjustably received in the second aperture. US2017156759 discloses a pectus excavatum correction apparatus that includes two pectus bars each made of a stick plate. Each bracket of the apparatus has a plate shape with a length-direction bent part. The first side of the bent part is hooked onto an upper or lower part of a rib and a second side of the bent part faces an outer surface of the pectus bar. Each of a first and second bridge has through-holes provided in two length-direction ends of the bridge. Each coupling unit includes a supporting part provided between the rib and the pectus bar, a slide protruding from an outer surface of the supporting part, and a second coupling part coupled to the first coupling part to press and fix the pectus bar, the bracket, and the bridge. The second distance is greater or less than the first distance within a preset range.

### BRIEF DESCRIPTION OF THE DRAWINGS

According to the invention, there is provided a pectus bar assembly as set out in claim 1. Additional features are set out in claims 2 to 14.

Associated methods are also described herein to aid understanding the invention. These methods do not form part of the claimed invention.

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates an X-ray image of a pectus bar assembly installed in a ribcage of a patient.
FIG. 2 illustrates an isometric view of a portion of a pectus bar assembly.
FIG. 3 illustrates an isometric view of a portion of a pectus bar assembly.
FIG. 3A illustrates an isometric view of a portion of a pectus bar assembly.
FIG. 4 illustrates an isometric view of a pectus bar assembly in a first configuration.
FIG. 5 illustrates an isometric view of a pectus bar assembly in a second configuration.
FIG. 6 illustrates an isometric view of a portion of a pectus bar assembly.
FIG. 7 illustrates an isometric view of a pectus bar assembly in a first configuration.
FIG. 8 illustrates an isometric view of a pectus bar assembly in a second configuration.
FIG. 9 illustrates an isometric view of a pectus bar assembly.
FIG. 10 illustrates an enlarged isometric view of a portion of a pectus bar assembly.
FIG. 11 illustrates a schematic view of a method.
FIG. 12 illustrates an isometric view of a portion of a pectus bar assembly.
FIG. 13 illustrates an isometric view of a portion of a pectus bar assembly.
FIG. 14 illustrates a side view of a portion of a pectus bar assembly.
FIG. 15 illustrates a side view of a portion of a pectus bar assembly.
FIG. 16 illustrates a side view of a portion of a pectus bar assembly.
FIG. 17 illustrates a side view of a portion of a pectus bar assembly.
FIG. 18 illustrates an isometric view of a pectus bar assembly.
FIG. 19 illustrates an isometric view of a portion of a pectus bar assembly.
FIG. 20 illustrates a cross-sectional view of a portion of a pectus bar assembly.
FIG. 21 illustrates an isometric view of a portion of a pectus bar assembly.
FIG. 22 illustrates an isometric view of a portion of a pectus bar assembly.
FIG. 23 illustrates an isometric view of a portion of a pectus bar assembly.

### DETAILED DESCRIPTION

In cases where pectus carinatum or pectus excavatum require surgical correction, a common corrective procedure includes securing a pectus bar to a patient's rib cage to reshape and stabilize the rib cage. This procedure can include the steps of: creating opposing incisions on each side of a patient's rib cage; inserting a curved pectus bar into one incision; weaving the pectus bar through ribs with the concave side of the bar facing the anterior side of the sternum; flipping the pectus bar to push against the anterior side of the sternum to expand the rib cage to a desired shape; securing the pectus bar to the rib cage; securing stabilizers to the pectus bar; securing the stabilizers to the rib cage; and, closing the incisions. Depending on the anatomy of the patient, current hardware solutions may be difficult to secure to a patient's ribs.

In some of these surgical procedures, two pectus bars are used, which can help to keep the sternum in a desired position. Hardware can be used to interconnect the pectus bars and to help prevent the bars from flipping following a procedure. However, the hardware can be relatively cumbersome and time consuming to install, while limiting adjustability. The devices and methods discussed herein can help to address these issues by including a pectus bar bridge that is simple to install and allows for easy adjustment of the pectus bars relative to each other.

For example, a pectus bar assembly can include a first pectus bar and a second pectus bar engageable with a sternum. The pectus bar assembly can also include a first bracket connectable to the first pectus bar and a second bracket connectable to the second pectus bar. The assembly can include a bridge connectable to the first bracket and the second bracket to allow the first bracket and the first pectus bar to translate along the bridge, and to allow the second bracket and the second pectus bar to translate, independently of the first pectus bar, along the bridge.

The above discussion is intended to provide an overview of subject matter of the present patent application. It is not intended to provide an exclusive or exhaustive explanation of the invention. The description below is included to provide further information about the present patent application.

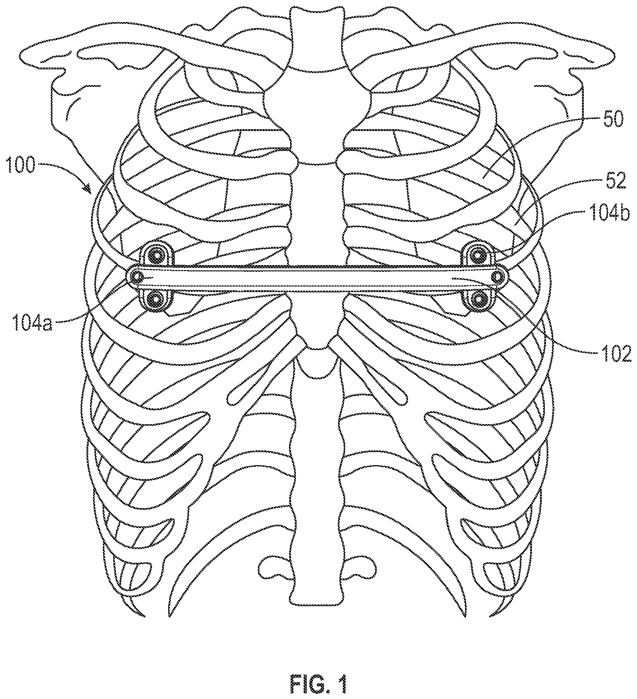

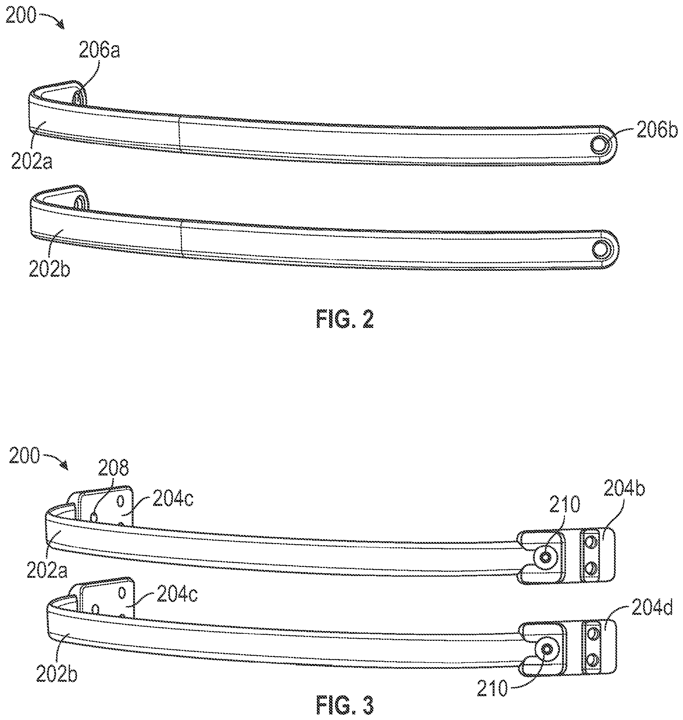

FIG. 1 illustrates an X-ray image of a pectus bar assembly 100, which can include a pectus bar 102 and stabilizers 104a and 104b. Also shown in FIG. 1 is ribcage 50, which includes ribs 52. FIG. 1 shows how the pectus bar 102 can be secured to a ribcage along with the stabilizers 104. FIGS. 2-10 discuss additional details of various pectus bar assemblies and procedures.

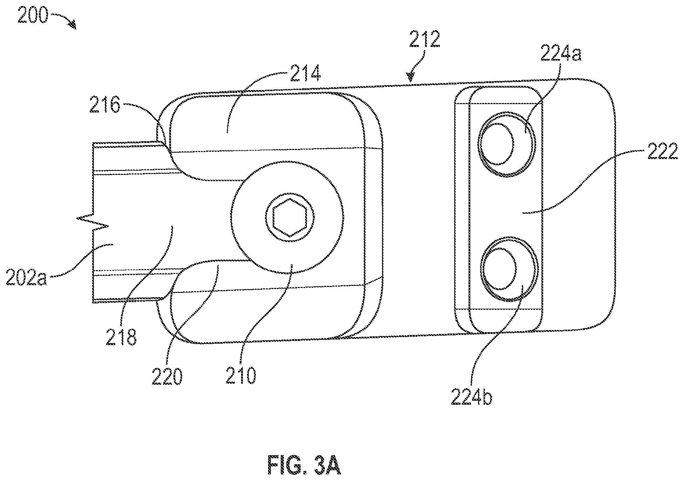

FIG. 2 illustrates an isometric view of a portion of a pectus bar assembly 200. FIG. 3 illustrates an isometric view of a portion of the pectus bar assembly 200. FIGS. 2 and 3 are discussed together below. The pectus bar assembly 200 can be similar to the pectus bar assembly 100 discussed above in that the pectus bar assembly 200 can be configured to be installed in a ribcage for correction of pectus excavatum or pectus carinatum. The pectus bar assembly 200 can differ in that the pectus bar assembly 200 can include multiple pectus bars and different hardware. Any of the pectus bar assemblies discussed above or below can be modified to include the features of the pectus bar assembly 200.

The pectus bar assembly 200 can include a pectus bar 202a (e.g., a first pectus bar) and a pectus bar 202b (e.g., a second pectus bar) (collectively referred to as pectus bars 202). Each of the pectus bars 202 can be a rigid or semi-rigid bar constructed of materials such as metals and plastics. For example, the pectus bars 202 can be constructed of biocompatible materials such as one or more of stainless steel alloys, cobalt-chromium, titanium, titanium alloys, polyether ether ketone (PEEK), polyether ketone ketone (PEKK), or the like.

The pectus bars 202 can have an elongate body forming a curve, such as a c-shape, and can be rigid (or semi-rigid) but flexible enough to be bent to match a curvature of a patient's ribcage using tools, such as a bar bender. As shown in FIG. 2, the pectus bars 202 can include bar bores 206a and 206b that can be located near terminations of the pectus bars 202 (e.g., the pectus bar 202a). The bar bores 206 can be configured to receive fasteners, such as bone screws or sutures, such as to secure the pectus bar 202 to ribs and/or soft tissues of ribcage 50. Optionally, the bar bores 206 can be threaded.

As shown in FIG. 3, the pectus bar assembly 200 can include stabilizers or brackets 204a-204d (collectively referred to as stabilizers 204). Stabilizers 204a and 204b can be connected to end portions of the pectus bar 202a and stabilizers 204c can be connected to end portions of the pectus bar 202b, as discussed in further detail below. Each of the stabilizers 204 can be a rigid or semi-rigid component constructed of materials such as metals and plastics. For example, the stabilizers 204 can be constructed of biocompatible such as one or more of stainless steel alloys, cobalt-chromium, titanium, titanium alloys, polyether ether ketone (PEEK), polyether ketone ketone (PEKK), or the like.

FIG. 3 also shows that each of the stabilizers 204 can include a bore 208 configured to receive a fastener 210 at least partially therein. The fastener 210 can extend at least partially into the bar bore 206b and the bore 208 to secure the stabilizers 204b to the pectus bar 202a. The bore 208 can be threaded to connect the fastener 210 to the bore 208 and therefore to the pectus bar 202 and the stabilizer 204. Any of the stabilizers 204 can include such a bore and can be securable to their respective bar bore 206 in a similar way. Further details of the stabilizers 204 are discussed below with respect to FIG. 3A.

FIG. 3A illustrates an isometric view of a portion of the pectus bar assembly 200. The pectus bar assembly 200 of FIG. 3A can be consistent with the pectus bar assembly 200 of FIGS. 2-3 discussed above. FIG. 3A shows additional details of the pectus bar assembly 200. For example, FIG. 3A shows that the stabilizer 204b can include a base 212 and a receiver 214 connected to the base 212. The receiver 214 can define an opening 216 configured to receive an end portion 218 of the pectus bar 202a at least partially therein, such as to align the bar bore 206b with the bore 208 of the stabilizers 204b. The receiver 214 can also include a slot 220. The slot 220 can be configured (e.g., sized or shaped) to allow the fastener 210 and the end portion 218 to be inserted into the receiver 214 when the fastener 210 is connected to the end portion 218 via the bore 206b, which can help to simplify the procedure.

The stabilizer 204b can also include a boss 222 that can extend from a surface of the base 212. The boss 222 define bores 224a and 224b that can be configured (e.g., threaded) to receive fasteners therein, as discussed below. As also discussed below, the boss 222 can be configured (e.g., sized or shaped) to be insertable into a bracket. Though the stabilizer 204b is discussed with respect to FIG. 3A, any of the stabilizers can include the components of the stabilizer 204b.

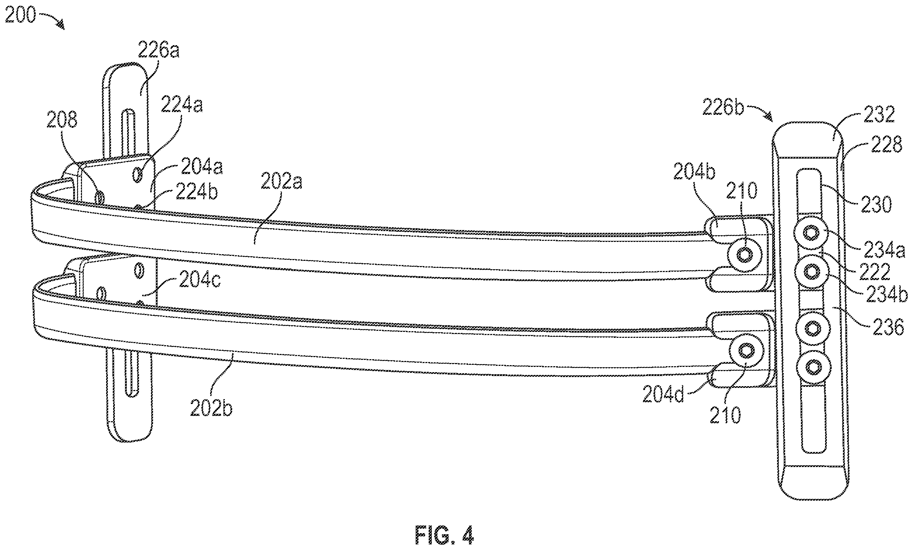

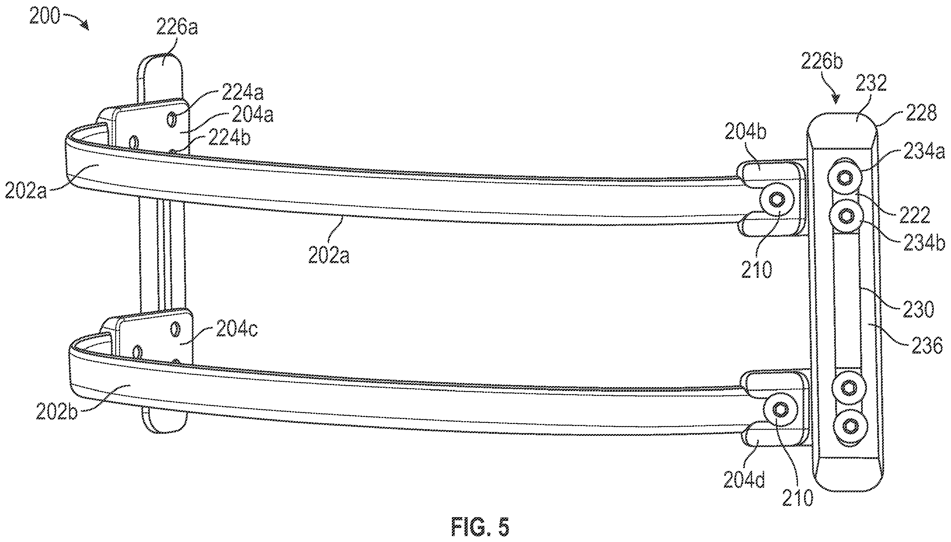

FIG. 4 illustrates an isometric view of the pectus bar assembly 200 in a first spacing configuration. FIG. 5 illustrates an isometric view of the pectus bar 200 assembly in a second spacing configuration. FIGS. 4 and 5 are discussed together below. The pectus bar assembly 200 of FIGS. 4-5 can be consistent with the pectus bar assembly 200 of FIGS. 2-3A discussed above. FIGS. 4-5 shows additional details of the stabilizers 204. Also, FIG. 4 shows the pectus bar assembly 200 adjusted to a spacing between the pectus bar 202a and the pectus bar 202b where one rib (e.g., one of the ribs 52) can be between the pectus bar 202a and the pectus bar 202b and FIG. 5 shows the pectus bar assembly 200 adjusted to a spacing between the pectus bar 202a and the pectus bar 202b where two ribs can be between the pectus bar 202a and the pectus bar 202b. FIGS. 4-5 also show additional components of the pectus bar assembly 200.

For example, FIGS. 4 and 5 show that the pectus bar assembly 200 can include a bridge 226a and a bridge 226b (collectively referred to as bridges 226). Each of the bridges 226 can include a body 228 defining a slot 230. The body 228 can include end portions 232 that can be tapered (or beveled or rounded), such as to help reduce palpability of the bridges following installation thereof. Each of the bridges 226 can be a rigid or semi-rigid component constructed of materials such as metals and plastics. For example, the bridges 226 can be constructed of biocompatible such as one or more of stainless steel alloys, cobalt-chromium, titanium, titanium alloys, polyether ether ketone (PEEK), polyether ketone ketone (PEKK), or the like.

The bridge 226b can be connectable to the stabilizers 204b and 204d to allow the stabilizer 204b and the first pectus bar 202a to translate along the bridge, and to allow the stabilizer 204d and the second pectus bar 202b to translate, independently of the first pectus bar 202a, along the bridge 226b. The bridge 226a can operate similarly to allow for independent adjustment or translation of the pectus bar 202a and the pectus bar 202b. The translation provided by the stabilizers of the pectus bars 202 is not limited or discrete. This can allow a surgeon to place the bars as desired or required by a procedure, whereas, many devices in the prior art includes holes at discrete increments to space apart pectus bars, which can limit options for placement in the pectus bars and result in interference with ribs.

The pectus bar assembly 200 can also include fasteners 234a and 234b (collectively referred to as fasteners 226). Each of the stabilizers 204 can receive fasteners 234a and 234b into bores 224a and 224b, respectively. The fasteners 234a and 234b can engage an outer surface 236 of the bridge 226b to secure the bridge 226b to the stabilizers 204b. The stabilizer 204d can be similarly connected to the bridge 226b and the stabilizers 204a and 204c can be similarly connected to the bridge 226a.

The slot 230 can be configured to receive at least a portion of the fasteners 234 therethrough to secure the stabilizer 204b to the bridge 226b. The slot 230 can also be configured to receive at least a portion of the boss 222 therein such that the boss 222 can be translatable within the slot 230 to enable translation of the stabilizer 204b along the bridge 226b. The boss 222 can be translatable within the slot 230 when the fasteners 234 are secured to the stabilizers 204b but not tightened against the outer surface 236 (or when the fasteners 234 are not secured to the stabilizers 204b), allowing for adjustment of the bars 202 during a procedure or allowing for connection of the stabilizers 204 to the bridges 226 at various locations.

For example, as shown in FIG. 4, the pectus bar 202a and the pectus bar 202b can be spaced apart from each other such that one rib can be located in between the bars 202a and 202b. However, as shown in FIG. 5, the stabilizers 204 can be connected to the bridges 226 such that two or more ribs can fit between the bars 202a and 202b. The stabilizers 204 can be translated between positions shown in FIGS. 4 and 5 when the stabilizers 204 are connected via the fasteners 234 and not tightened, as discussed above. When the fasteners 234a and 234b are secured to the bores 224a and 224b, respectively, and are tightened to engage the outer surface 236, movement of the stabilizers 204b with respect to the bridge 226b can be limited. Also, engagement by the boss 222 with ends of the slot 230 can limit translation of the stabilizers 204b with respect to the bridge 226b.

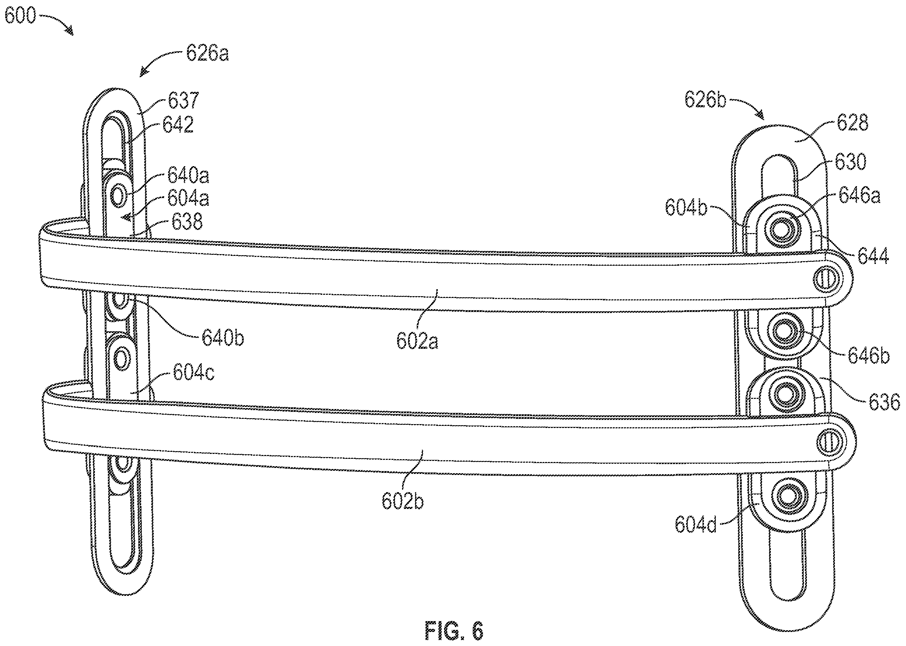

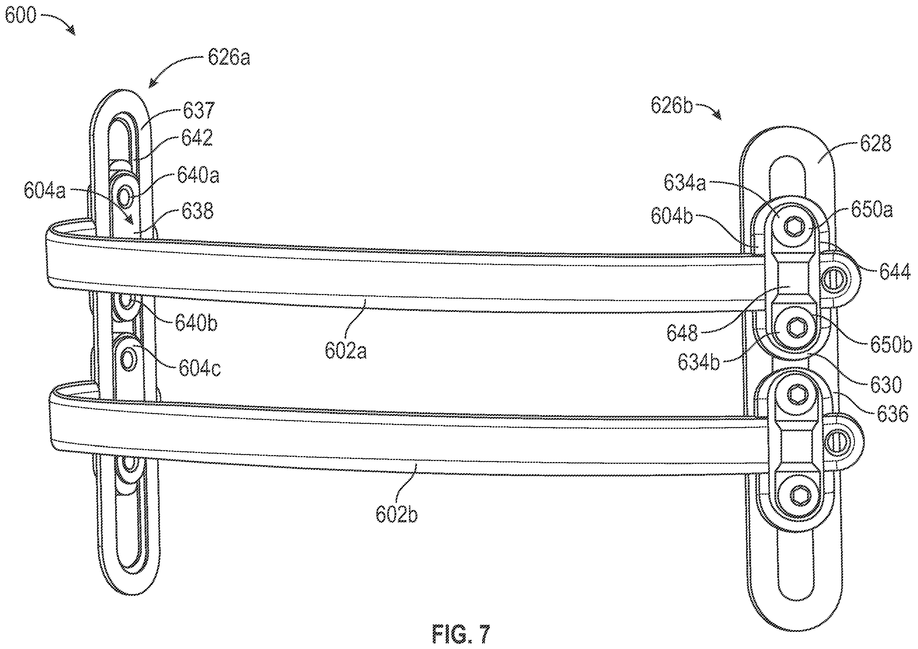

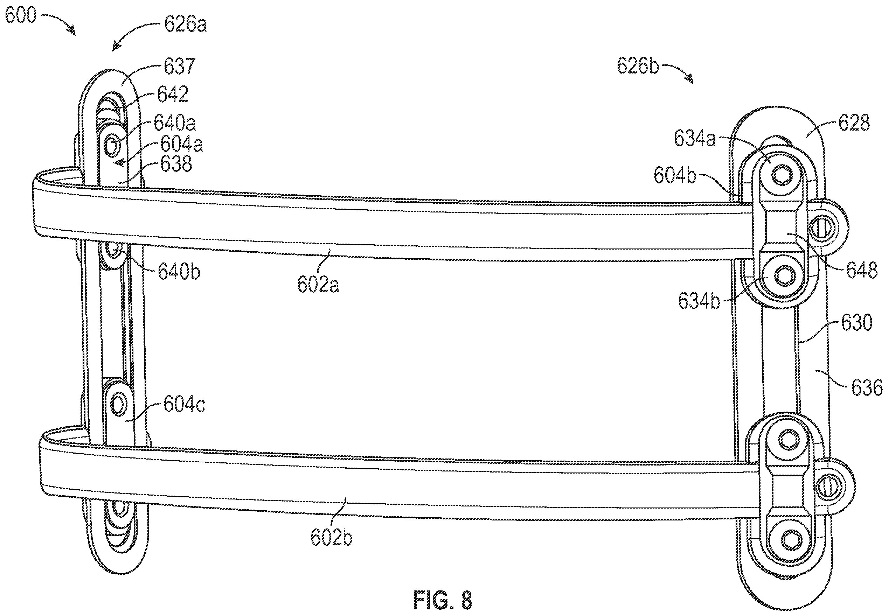

FIG. 6 illustrates an isometric view of a portion of a pectus bar assembly 600. FIG. 7 illustrates an isometric view of the pectus bar assembly 600 in a first configuration. FIG. 8 illustrates an isometric view of the pectus bar assembly 600 in a second configuration. FIGS. 6-8 are discussed together below. The pectus bar assembly 600 can be similar to the pectus bar assemblies discussed above. The pectus bar assembly 600 can include hardware for adjusting the bars vertically or the bridges horizontally. Any of the pectus bar assemblies discussed above or below can be modified to include the features of the pectus bar assembly 600.

The pectus bar assembly 600 can include pectus bars 602a and 602b, which can be similar to the bars 202a and 202b, discussed above. The pectus bar assembly 600 can also include stabilizers 604a-604d. The stabilizers 604a and 604b can be configured to connect to the pectus bar 602a and the stabilizers 604C and 604D can be configured to connect to the pectus bar 602b.

Each of the stabilizers 604 can include a base 638, as shown most clearly in FIG. 6. Each base 638 can include bores 640a and 640b that can be configured to receive fasteners 634a and 634b, respectively, at least partially therein, as shown in FIGS. 7-8. For example, the bores 640a and 640b can be threaded to receive screws or bolts. The base 638 can be configured to engage a first side (or back side or posterior side) 637 of the bridge 626. The base can also be insertable into a recess 642 of the bridge 626, which can at least partially surround a slot 630 of the bridge 626.

Each of the stabilizers 604 can also include a plate 644, as shown most clearly in FIG. 6. The plate 644 can engage a second side 636 (or front side or anterior side) of the bridge 626 and can connect to the plate 644, such as through a snap interface. A portion of the base 638 or a portion of the plate 644 can extend at least partially through the slot 630 to secure the base 638 and the plate 644 to the bridge 626. When the base 638 and the plate 644 are connected to the bridge 626, the base 638 and the plate 644 can be translatable along the slot 630. The plate 644 can also include bores 646a and 646b that can be aligned with the bores 640a and 640b of the base 638 when the base 638 is connected to the plate 644. As shown in FIG. 6, the plate 644 can engage a first side of the pectus bar 602a.

As shown in FIGS. 7 and 8, each of the stabilizers 604 can include a cap 648. The cap 648 can be engageable with the pectus bar 602a and can be securable to the plate 644. The cap 648 can include bores 650a and 650b configured to receive fasteners 634a and 634b at least partially therein or therethrough. For example, at least a portion of the fasteners 634a and 634b can extend through bores 650a and 650b of the cap 648, respectively, and through bores 646a and 646b of the plate 644, respectively, and into the bores 640a and 640b of the base 638, respectively, to secure the cap 648, the plate 644, and the base 638 to each other.

When the fasteners 634a and 634b are secured to the cap 648, the plate 644, and the base 638, but not tightened, the cap 648, the plate 644, the base 638, and the pectus bar 602a can be translatable along the bridge 626b. For example, as shown in FIG. 7, the pectus bar 602a and the pectus bar 602b can be spaced apart from each other such that one rib can be located in between the bars 602a and 602b. As shown in FIG. 8, the stabilizers 604 can be translated along the bridges 626a and 626b such that two or more ribs can fit between the bars 602a and 602b. That is, the stabilizers 604 can be translated between the positions shown in FIGS. 7 and 8 when the cap 648, the base 638, and the plate 644 are connected and the fasteners 634 are not tightened.

Also, when the fasteners 634a and 634b are secured to the cap 648, the plate 644, and the base 638, but the fasteners 634a and 634b are not tightened, the cap 648, the plate 644, the base 638, and the bridge 626b can be translatable along the pectus bar 602a. And, the bridges 626 and stabilizers 604 can be translated along the bars 602 when the fasteners 634 are connected to the cap 648 and the base 638 but are not tightened. When the fasteners 634a and 634b are tightened to the cap 648, the plate 644, and the base 638, translation of the stabilizers 604 (and the bars 602) with respect to the bridges 626 can be limited or prevented and translation of the stabilizers 204 and the bridges 626 with respect to the bars 602 can be limited or prevented. In this way, the assembly 600 can allow for intraoperative adjustment of the hardware as necessary to fit an individual patient.

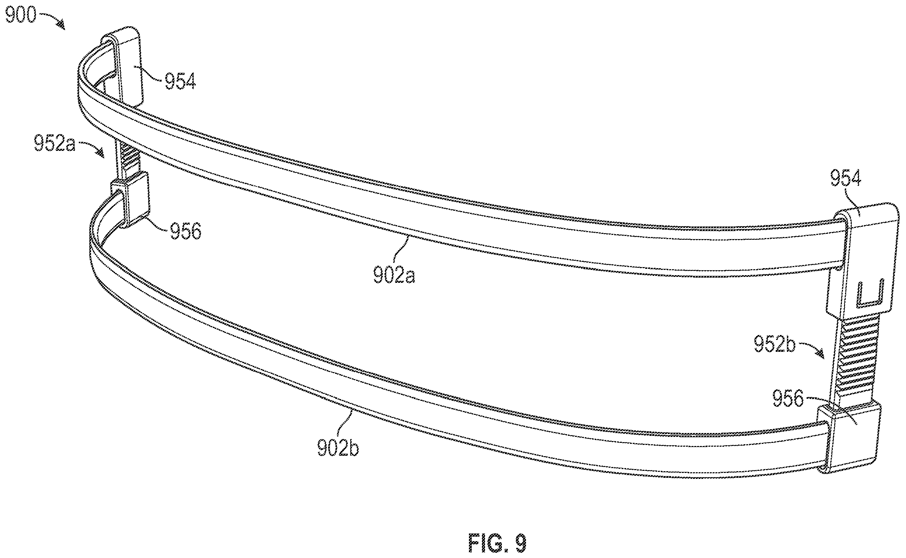

FIG. 9 illustrates an isometric view of a pectus bar assembly 900. The pectus bar assembly 900 can be similar to the pectus bar assemblies discussed above. The pectus bar assembly 900 can include hardware for quickly securing the pectus bars to each other. Any of the pectus bar assemblies discussed above or below can be modified to include the features of the pectus bar assembly 900.

The pectus bar assembly 900 can include a pectus bar 902a and a pectus bar 902b, which can be similar to the pectus bars discussed above. The pectus bar assembly 900 can also include a stabilizer assembly 952a and a stabilizer assembly 952b. Each of the stabilizer assemblies can include a first bracket 954 and a second bracket 956. The first brackets 954 can be connected to the pectus bar 902a and the second brackets 956 can be connected to the pectus bar 902b. Optionally, one of the first brackets 954 and one of the second brackets 956 can be connected to the pectus bar 902a and one of the first brackets 954 and one of the second brackets 956 can be connected to the pectus bar 902b. That is one of the stabilizer assemblies 952 can be connected to the bars 902 in a configuration opposite the other.

Each of the first brackets 954 and the second brackets 956 can be a rigid or semi-rigid components constructed of materials such as metals and plastics. For example, the first brackets 954 and the second brackets 956 can be constructed of biocompatible such as one or more of stainless steel alloys, cobalt-chromium, titanium, titanium alloys, polyether ether ketone (PEEK), polyether ketone ketone (PEKK), or the like.

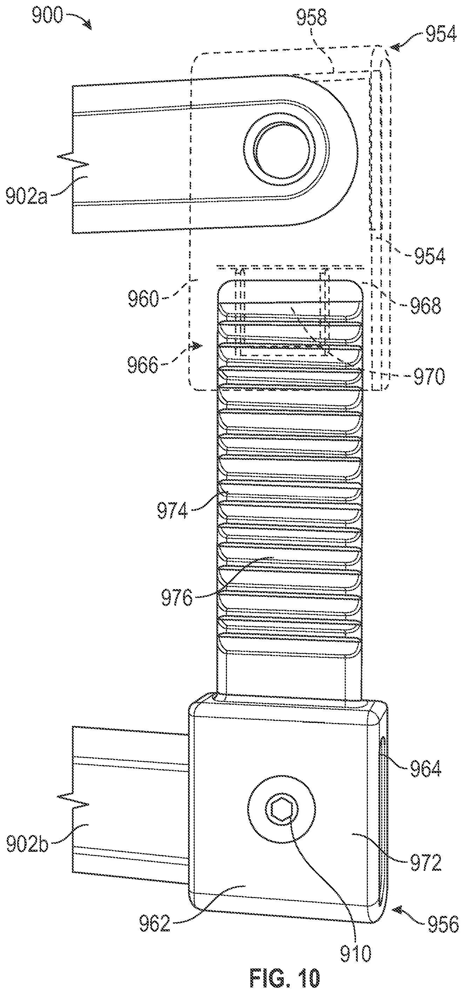

FIG. 10 illustrates an enlarged isometric view of a portion of the pectus bar assembly 900. The pectus bar assembly 900 of FIG. 10 can be consistent with the pectus bar assembly 900 of FIG. 9; FIG. 10 shows additional details of the pectus bar assembly 900. For example, FIG. 10 shows that the first bracket 954 can include a bar slot 958, which can be a recess, opening, or bore extending at least partially into a body 960 of the first bracket 954. The bar slot 958 can extend entirely through the body 960 such as to allow the pectus bar 902a to pass through the first bracket 954. Optionally, the bar slot 958 can extend only partially into the body 960, such as to limit movement of the pectus bar 902a with respect to the first bracket 954.

Similarly, the second brackets 956 can include a bar slot 964, which can be a recess, opening, or bore extending at least partially into a body 962 of the second bracket 956. The bar slot 964 can extend entirely through the body 962 such as to allow the pectus bar 902b to pass through the second bracket 956. Optionally, the bar slot 964 can extend only partially into the body 962, such as to limit movement of the pectus bar 902b with respect to the second bracket 956.

The first bracket 954 can also include a receiver 966 defining a slot 968 extending at least partially into the body 960. The slot 968 can terminate within the body 960 prior to interesting the bar slot 958. The slot 968 can be orthogonal to the bar slot 958 or can be in an orientation not parallel to the bar slot 958. The first bracket 954 can also include a pawl 970 connected to the receiver 966. The pawl 970 can include one or more projections extending at least partially into the slot 968. The pawl 970 can be configured to reversibly deflect. The pawl 970 can be integrally formed into the body 960, such as via a living hinge. Optionally, the pawl 970 can be connected to the body 960.

The second bracket 956 can include a projection 974 extending from a body 972 of the second bracket 956. The projection 974 can be insertable into the receiver 966 to connect the first bracket 954 to the second bracket 954 and to secure the first pectus bar 902a to the second pectus bar 902b. The projection 974 and the receiver 966 can together form a ratcheting interface to secure the first bracket 954 to the second bracket 956. For example, the projection 974 can include a plurality of teeth 976, which can be engageable with the pawl 970 when the projection 974 is inserted into the receiver 966. The pawl 970 can engage the teeth 976 and deflect when the projection 974 moves into the receiver and the pawl 970 can engage and retain one or more of the teeth 976 to limit movement of the projection 974 out of the receiver 966. In this way, the receiver 966 and the projection 974 can be relatively quickly and securely connected to secure the pectus bar 902a to the pectus bar 902b.

Also, optionally, the projection 974 can be engageable with the slot 968 to limit translation of the projection 974 into the slot. For example, the projection 974 can be measured and cut to a desired length prior to insertion such that engagement between the projection 974 and the slot 968 can be used to position the bars pectus bar 902a and pectus bar 902b with respect to each other.

Optionally, the pectus bar assembly 900 can include one or more fasteners, such as a fastener 910, which can be threadably securable to a bore of the second bracket 956 and can be insertable through a bore of the pectus bar 902b, such as to limit translation of the pectus bar 902b with respect to the second bracket 956. The first bracket 954 can optionally be similarly configured.

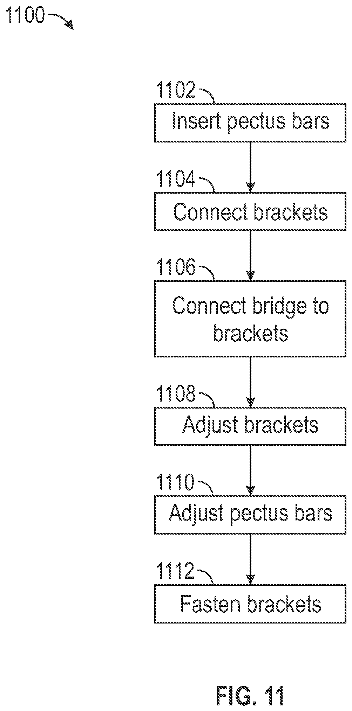

FIG. 11 illustrates a schematic view of the method 1100, in accordance with at least one example of this disclosure. The method 1100 can be a method of installing a pectus bar. More specific examples of the method 1100 are discussed below. The steps or operations of the method 1100 are illustrated in a particular order for convenience and clarity; many of the discussed operations can be performed in a different sequence or in parallel without materially impacting other operations. The method 500 as discussed includes operations performed by multiple different actors, devices, and/or systems. It is understood that subsets of the operations discussed in the method 500 can be attributable to a single actor, device, or system could be considered a separate standalone process or method.

The method can begin at step 1102, which can be inserting a first pectus bar and a second pectus bar into a thoracic cavity, such as the pectus bar 202a and the pectus bar 202b. At step 1104, a first bracket can be connected to the first pectus bar and a second bracket can be connected to the second pectus bar. For example, the stabilizer 204b can be connected to the pectus bar 202a and the stabilizer 204d can be connected to the stabilizers 204b.

At step 1106, a bridge can be connected to the first bracket and the second bracket. For example, the bridge 226b can be connected to the stabilizers 204b and 204d. At step 1108, the first and second bracket can be adjusted with respect to the bridge. For example, the stabilizers 204b or 204d can be translated along the bridge 226b. At step 1110, the pectus bars can be adjusted with respect to the bridges. For example, the pectus bar 602a and the pectus bar 602b can be adjusted with respect to the stabilizers 604a-604d by translating the stabilizers (and the bridges 626) along the pectus bars 602. At step 1114, the brackets can be fastened. For example, the stabilizers 204 can be fastened or otherwise secured to the bridges 226, such as to secure the pectus bars 202 to the bridges 226. Additional steps discussed above with respect to FIGS. 2-10 can be incorporated into the method 1100.

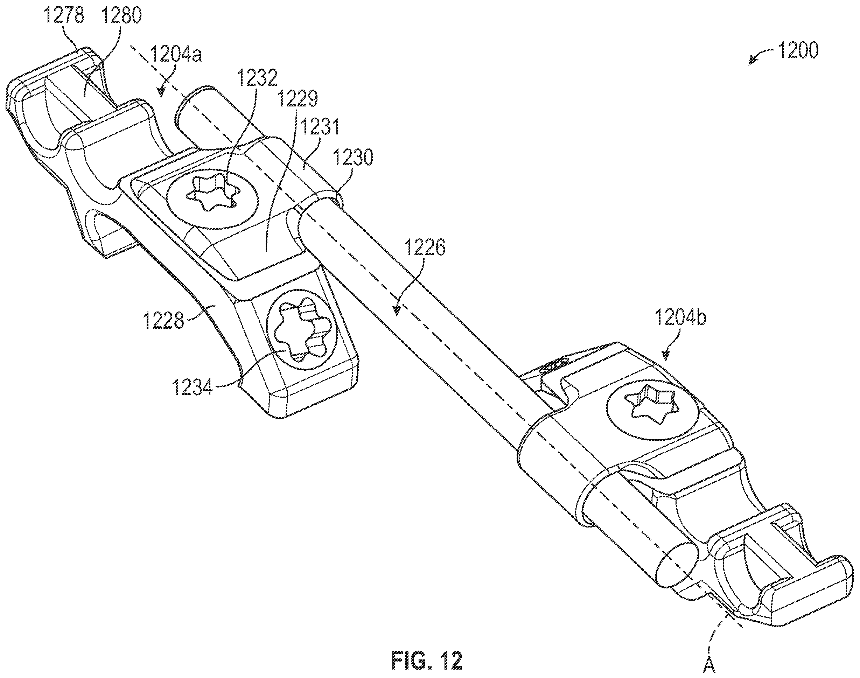

FIG. 12 illustrates an isometric view of a portion of a pectus bar assembly 1200. The pectus bar assembly 1200 can be similar to the pectus bar assemblies discussed above or below. The pectus bar assembly 1200 can include bar links securable to pectus bars and securable to a bridge to help stabilize the assembly following implantation.

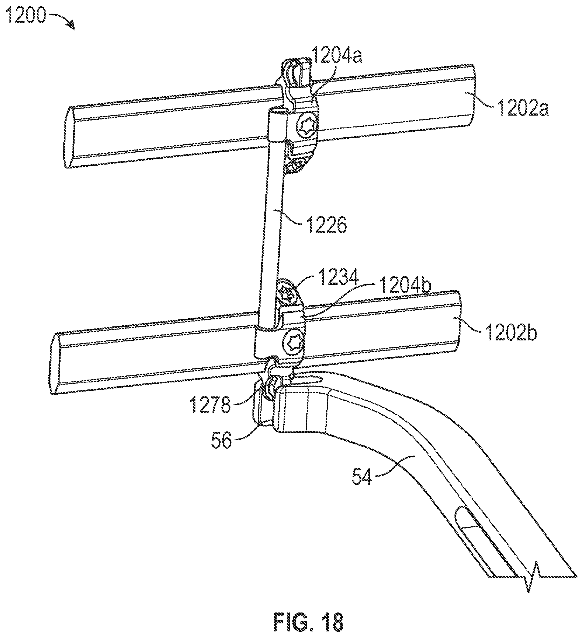

The pectus bar assembly 1200 can include a first pectus bar 1202a and a second pectus bar 1202b (both shown in FIG. 18) and a first bar link 1204a and a second bar link 1204b. The pectus bar assembly 1200 can also include a bridge 1226. Though the pectus bar assembly 1200 is discussed as including two pectus bars and two bar links, the pectus bar assembly 1200 can include 1, 2, 3, 4, 5, 6, or the like pectus bars, and can include 1, 2, 3, 4, 5, 6, or the like bar links (or stabilizers or brackets).

The pectus bars 1202 can be similar to the pectus bars discussed above (e.g., the 202) in that the pectus bars 1202 can be a rigid or semi-rigid bar constructed of one or more biocompatible materials such as metals and plastics. For example, the pectus bars 1202 can be constructed of biocompatible materials such as one or more of stainless-steel alloys, cobalt-chromium, titanium, titanium alloys, polyether ether ketone (PEEK), polyether ketone ketone (PEKK), or the like. The pectus bars 1202 can have an elongate body forming a curve, such as a c-shape, and can be rigid (or semi-rigid) but flexible enough to be bent to match a curvature of a patient's ribcage using tools, such as a bar bender.

The bar links 1204a and 1204b can each be connectable to portions of the pectus bars 1202a and 1202b, respectively, and the bar links 1204a and 1204b can be translatable along the pectus bars 1202a and 1202b, respectively, as discussed in further detail below. Each of the bar links 1204a and 1204b can be a rigid or semi-rigid component constructed of materials such as metals and plastics. For example, the bar links 1204a and 1204b can be constructed of biocompatible such as one or more of stainless-steel alloys, cobalt-chromium, titanium, titanium alloys, polyether ether ketone (PEEK), polyether ketone ketone (PEKK), or the like. The bar links 1204a and 1204b can be the same configuration but rotated; in other examples the bar links 1204a and 1204b can be mirror copies of each other.

The bridges 1226 can be connected to the pectus bars 1202 by the bar links 1204 such as to reinforce the pectus bars 1202 as an assembly. Each of the bridges 226 can be a rigid or semi-rigid component constructed of materials such as metals and plastics. For example, the bridges 226 can be constructed of biocompatible such as one or more of stainless steel alloys, cobalt-chromium, titanium, titanium alloys, polyether ether ketone (PEEK), polyether ketone ketone (PEKK), or the like. The bridges can be cylindrical in shape but can have other shapes such as a rectangular prism, hexagonal prism, octagonal prism, or the like.

Each of the bar links 1204 can include a body 1228 and an arm 1229 connected to the body 1228. The body 1228 and the arm 1229 can together form a slot 1230 to receive the bridge 1226 at least partially therein or therethrough. The arm 1229 can be connected to the body 1228 by a living hinge 1231 and the arm 1229 and the body 1228 can together form a clamp biased to an open position by the living hinge 1231. The living hinge 1231 can be integral to the body 1228 and the arm 1229, and the living hinge 1231 can at least partially form the slot 1230. The living hinge 1231 can be configured to elastically deform, flex, or move to enable relative movement of the arm 1229 with respect to the body 1228 which can cause a change in size of the slot 1230.

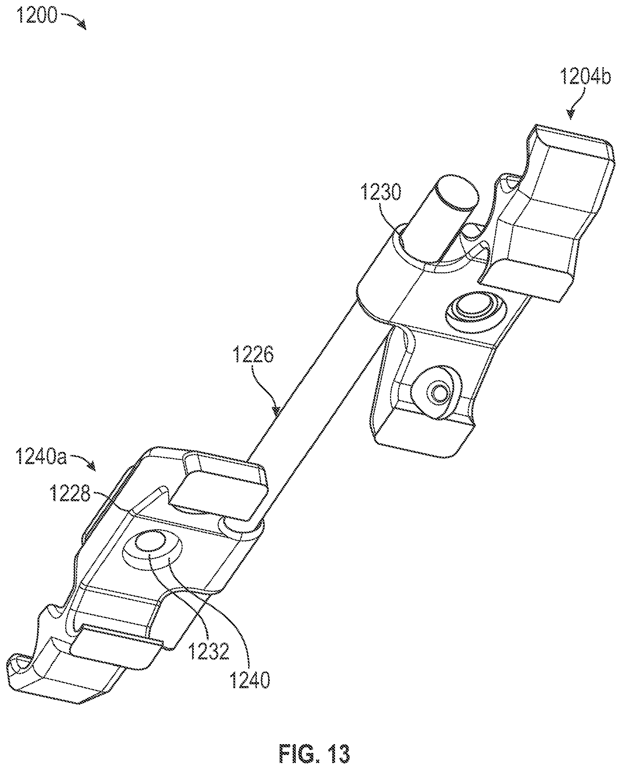

The bar links 1204 can also include an arm actuator 1232 that can be connected to the body 1228 and to the arm 1229. The arm actuator 1232 can extend through the arm 1229 and can threadably engage a bore 1240 (shown in FIG. 13) of the body 1228, where the bore 1240 can be threaded. The arm actuator 1232 can be a set screw, cam, screw, bolt, fastener or the like that can be operable to move between a first position where the bridge 1226 is movable with respect to the body 1228 and a second position where the arm 1229 and the body 1228 engage the bridge 1226 to clamp the bridge 1226 to secure the bar links 1204 to the bridge 1226. Because the arm 1229 is connected to the body 1228 by the living hinge 1231, when the arm actuator 1232 causes the arm 1229 to move, the living hinge 1231 can allow the arm 1229 to elastically deflect with respect to the body 1228. The arm 1229 can also be biased by the living hinge 1231 to an open or partially open position. Each of the bar links 1204 can also include an actuator 1234 that can be connected to the body 1228 and can be operable to secure the bar links 1204 to pectus bars, as discussed in further detail below.

When the arm 1229 (e.g., of the bar link 1204a) is in the open or unlocked position, the bar link 1204a can be translatable or movable along an axis A of the bridge 1226. The bar link 1204a can also be rotatable about the bridge 1226 (e.g., about the axis A of the bridge 1226). The bar links 1204 be independently adjustable along the bridge 1226 and around the bridge 1226. Optionally, if the bar links 1204 are fixed to the pectus bars, the bridge 1226 can be adjusted relative to the bar links 1204. The translation and rotation provided by the bar links 1204 is not limited or discrete. This can allow a surgeon to place the bars as desired or required by a procedure, whereas many devices in the prior art include holes at discrete increments to space apart pectus bars, which can limit options for placement in the pectus bars and can result in interference with ribs.

FIG. 12 also shows that the bar links 1204 can each include a head 1278 that can be connected to the body 1228. The head 1278 can be used by a physician to position the bar links 1204 with respect to anatomy, the pectus bars 1202, or the bridge 1226. The head 1278 can also include a rib 1280 that can be configured to be grasped by an instrument, such as a needle holder or forceps, to maneuver or position the bar links 1204, which can be helpful for positioning with respect to the bridge 1226 when the bridge 1226 is a cylindrical rod, as shown in FIG. 12. The head 1278 can also be used to at least partially deform the body 1228 for extraction or explantation of one or more components of the pectus bar assembly 1200, as discussed in further detail below. The head 1278 can be lower than (e.g., posterior of) the arm 1229 to help limit or reduce palpability of the head 1278.

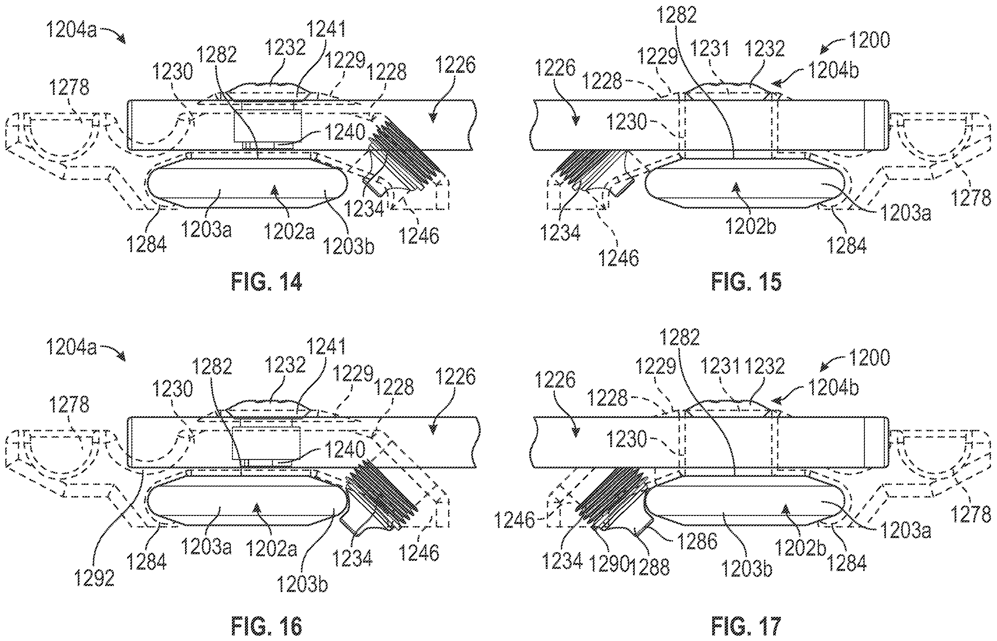

FIG. 14 illustrates a side view of a portion of the pectus bar assembly 1200. FIG. 15 illustrates a side view of a portion of the pectus bar assembly1200. FIG. 16 illustrates a side view of a portion of the pectus bar assembly 1200. FIG. 17 illustrates a side view of a portion of the pectus bar assembly 1200. FIGS. 14-17 are discussed together below. The pectus bar assembly 1200 of FIGS. 14-17 can be consistent with FIGS. 12 and 13 above; FIGS. 14-17 show additional details of the pectus bar assembly 1200. For example, FIGS. 14-17 show the pectus bars 1202a and 1202b and how the bar links 1204a and 1204b can connect to the first pectus bars 1202a and 1202b, respectively.

FIGS. 14-17 also show additional details of the bar links 1204. For example, FIGS. 14-17 show that each of the bodies 1228 of the bar links 1204 can include or can define an opening 1282 that can be configured to receive at least a portion of the pectus bar 1202 therein. The opening 1282 can be at least partially defined by a shelf 1284 of the body. The shelf 1284 can be shaped to conform to either end portion 1203a or 1203b of the first pectus bar 1202a or the second pectus bar 1202b. That is, the shelf 1284 can receive, for example, the end portion 1203a at least partially therein when the first pectus bar 1202a is inserted into the opening 1282, as shown in FIGS. 14-17. The shelf 1284 and the actuator 1234 can be together shaped and sized to allow the bar links 1204 to be placed onto the pectus bars 1202 from above (or anteriorly).

FIGS. 14-17 shows further details of the bore 1240 of the arm actuator 1232, which can extend at least partially through the body 1228. Also, the arm 1229 can include an actuator bore 1241 that can extend at least partially through the arm 1229 and can be aligned (e.g., coaxial with) the bore 1240. An upper portion of the actuator bore 1241 can be tapered or otherwise angled to be complementary to an underside of a head of the arm actuator 1232 such that the arm actuator 1232 cannot pass through the arm 1229 towards the body 1228. The body 1228 can include a swage of the bore 1240 in the body 1228 that can be configured, together with the head and the actuator bore 1241, to help captivate or capture the arm actuator 1232. Optionally, the bore 1240 can be threaded to engage with a threaded portion of the arm actuator 1232 such that when the arm actuator 1232 is rotated (e.g., clockwise), the arm actuator 1232 moves into the body 1228, bringing the arm 1229 closer to the body 1228, closing the slot 1230 and clamping down onto the bridge 1226 to limit rotation of the bar link 1204a with respect to the bridge 1226 and limiting translation of the bar link 1204a along the bridge 1226. The bar link 1204b can be similarly configured or operated.

FIGS. 14-17 also show an actuator 1234 located at least partially within a bore 1246 of the body 1228 of each of the bar links 1204. The actuator 1234 can be a set screw, cam, screw, bolt, fastener or the like that can be operable to move between a first position (shown in FIGS. 14 and 15) where the pectus bar(s) 1202 are movable with respect to the body 1228 and between a second position where the actuator 1234 engage the pectus bar(s) 1202 to clamp the bar links 1204 to the pectus bar(s) 1202. As shown in FIGS. 14 and 15, the actuator 1234 can be in a fully retracted position, which can allow, for example, the pectus bar 1202a to be inserted into the opening 1282 of the bar link 1204a. The actuator 1234 can be translated relative to the body 1228 by rotating the actuator 1234 with respect to the body 1228, such as with a cam, screw, or bolt.

The actuator 1234 can be limited or prevented from moving beyond the retracted position by a stop, swage, or the like of the actuator 1234, which can be configured to help capture or captivate the actuator 1234 at least partially within the bore 1246. During assembly, the actuator 1234 can be threaded into the bore 1246 from the opening 1282. Following insertion, the actuator 1234 can be swaged near its proximal side (or anterior side, such as away from the opening 1282). Similarly, the actuator 1234 can be limited or prevented from moving beyond the extended position by a stop, such as a thread run-out at a distal portion of the actuator 1234 or other stop of the bore 1246 or the actuator 1234.

As shown in FIGS. 16 and 17, the actuators 1234 can be operated (e.g., rotated through a threaded portion of the bore 1246) to engage a second end portion 1203b of the pectus bars 1202 to cause the first end portion 1203a to engage the shelf 1284 opposite the actuator 1234, thereby securing the bar links 1204 to the pectus bars 1202. Such engagement can be reversed by reversing (e.g., rotating in a counter-clockwise direction) the actuator 1234 to release the bar links 1204 from the pectus bars 1202.

As shown in FIG. 17, the actuator 1234 can be shaped such that it has a flat distal tip 1286 connected to a curved, arced, or radiused portion 1288 that is connected to a body 1290, which can be a threaded portion (e.g., threaded portion 1290). The radiused portion 1288 can be configured to engage a curved surface or portion of the second end portion 1203b. The arc or radius of curvature of the radiused portion 1288 can be complementary (e.g., shaped to receive or conform to) the end portion 1203b. The radius of curvature of the radiused portion 1288 can be between 0.5 millimeters (mm) and 2.5 mm, such as between 1 mm and 2mm. For example, the radius of curvature can be 1.1 mm, 1.2 mm, 1.3 mm, 1.4 mm, 1.5 mm, 1.6 mm, 1.7 mm, 1.8 mm, 1.9 mm, or the like. Also, the distal tip 1286 can have a diameter of between 0.5 mm and 2.5 mm, such as between 1 mm and 2mm. For example, the diameter of the distal tip 1286 can be 1.1 mm, 1.2 mm, 1.3 mm, 1.4 mm, 1.5 mm, 1.6 mm, 1.7 mm, 1.8 mm, 1.9 mm, or the like. In one example, the distal tip can be about 1.6 mm and the radius of curvature can be about 1.65 mm.

The flat distal tip 1286 and the radiused portion 1288 can also together be shaped such that the flat distal tip 1286 does not engage the end portion 1203b as the actuator 1234 extends from its retracted position to the extended position when the pectus bar 1202a is in the opening 1282, which could result in a force applied to the pectus bar 1202b that forces the pectus bar 1202b out of the opening 1282. Instead, the flat distal tip 1286 and the radiused portion 1288 can be shaped such that as the actuator 1234 extends into the opening 1282, the flat distal tip 1286 does not engage the second pectus bar 1202b and only the radiused portion 1288 engages the end portion 1203b to apply a force towards the body 1228 or the shelf 1284, helping to simplify installation of the pectus bar assembly 1200.

FIGS. 14-17 also show that the head 1278 can extend from the body 1228 away from the actuator 1234 and the head 1278 can be connected to a body 1228 on the same side as the shelf 1284. The body 1228 can also include a recess 1292 (shown in FIG. 16) that can create a relatively thin portion of the body 1228 between the recess 1292 and the shelf 1284, which can be used as discussed in FIG. 18 below.

FIG. 18 illustrates an isometric view of the pectus bar assembly 1200. The pectus bar assembly 1200 of FIG. 18 can be consistent with FIGS. 12-17 discussed above. FIG. 18 shows how the head 1278 can be used. For example, during an extraction, removal or explantation of one or more components of the pectus bar assembly 1200, it may be required to remove the bar link 1204b from the second pectus bar 1202b, which can require that the actuator 1234 be actuated to disengage the second pectus bar 1202b. However, tissue or bone can grow over or into the recess of the actuator 1234 that is configured to receive a tool or instrument, such as a hexolubular interface, hexagonal interface, cross-recess interface, or the like. In such a situation, it may be difficult to insert the instrument into the interface or can be time consuming to clear debris. When this occurs, the head 1278 can be inserted into an opening 56 of a bender 54 and the bender 54 can be operated to deform the body 1228 such that the body 1228 bends about the recess 1292 (shown in FIG. 16) and the opening 1282 increases in size to allow the bar links 1204b to be removed from the second pectus bar 1202b without operating the actuator 1234. The head 1278 of the bar links 1204a can be similarly manipulated to free the bar links 1204a from the first pectus bar 1202a allowing for explantation of the pectus bar assembly 1200 without actuation of the actuator 1234.

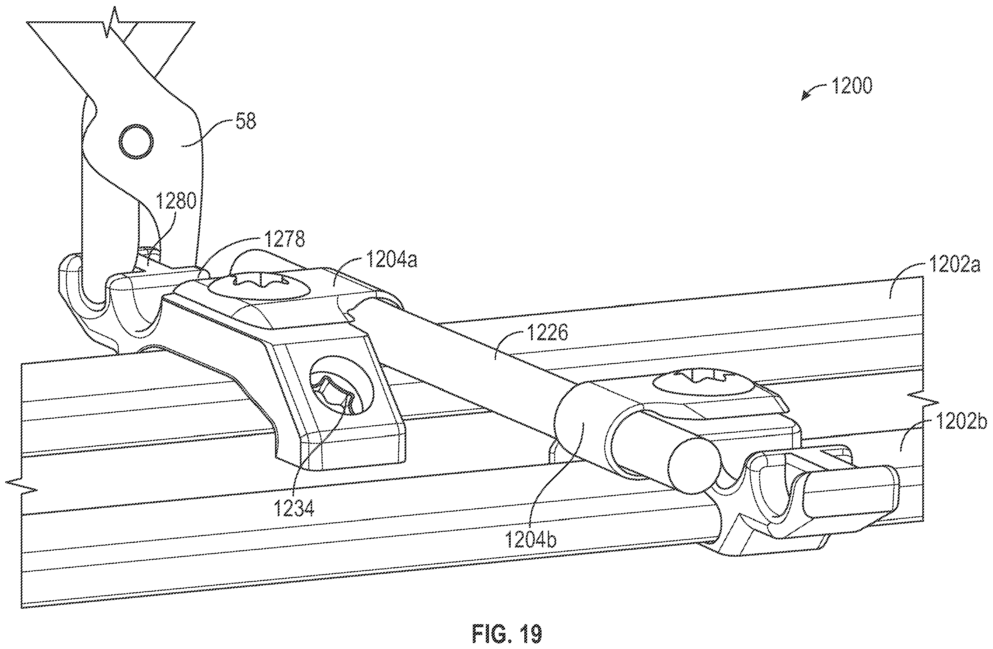

FIG. 19 illustrates an isometric view of a portion of the pectus bar assembly 1200. The pectus bar assembly 1200 of FIG. 19 can be consistent with FIGS. 12-18 discussed above. FIG. 19 shows other ways that the head 1278 can be used. For example, during installation or during extraction, it may be desired to manipulate or position the bar link 1204a with respect to the first pectus bar 1202a, the bridge 1226, or anatomy. In such a case, a grasping instrument 58 (e.g., an instrument including jaws, such as a needle holder or forceps) can be used to grasp the rib 1280 allowing a physician or surgeon to position the bar links 1204a easily using the instrument 58, such as along the bridge 1226 or around the bridge 1226.

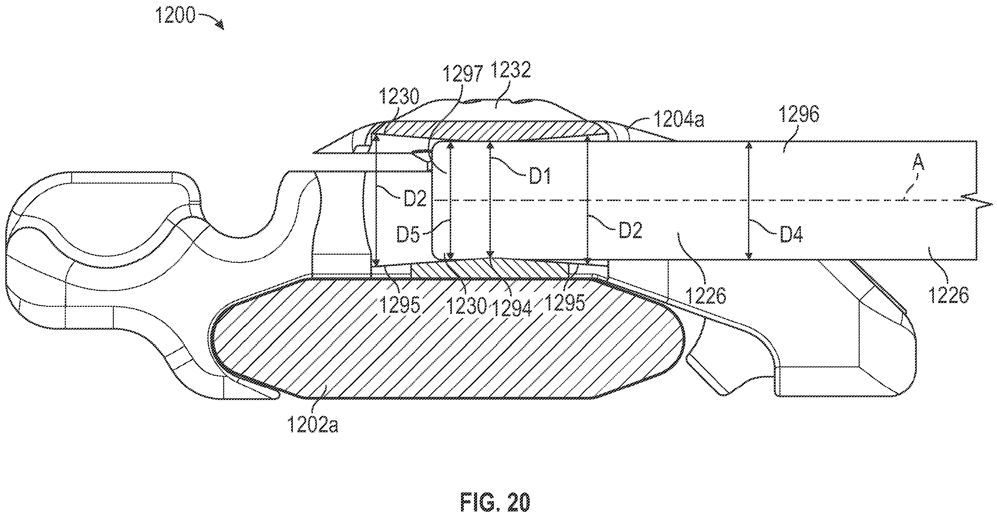

FIG. 20 illustrates a cross-sectional view of a portion of the pectus bar assembly 1200. The pectus bar assembly 1200 of FIG. 20 can be consistent with FIGS. 12-19 discussed above. FIG. 20 shows how the bridge 1226 can interact with the slot 1230. More specifically, the slot 1230 can include a primary bore 1294 and angled portions 1295 where the primary bore 1294 can define a diameter D1 and the tapered portions can define a diameter D2 that is larger than the diameter D1. The angled portions 1295 can be tapered or angled and can extend from the primary bore 1294, which can be near a center of the slot 1230, towards respective ends of the slot 1230.

The bridge 1226 can include a shaft 1296 having a diameter D4 along a length of the shaft 1296, which can be a majority of a length of the bridge 1226. The bridge 1226 can also include a flared or enlarged portion 1297 located at an end of a shaft 1296. The bridge 1226 can include an enlarged portion at each of its ends. The enlarged portion 1297 can have or define a diameter D5 that is larger than a diameter D4 of the shaft 1296. This enlarged diameter can be slightly larger than the diameter D1 such that when the bridge 1226 moves through the slot 1230, there is some resistance to insertion or removal of the bridge 1226 into or from the slot 1230.

Also, because the diameters D2 can be larger than the diameter D4 of the shaft 1296 and because the diameter D1 can be only slightly larger than the diameter D4 of the shaft 1296, the bar links 1204 can be configured to tilt in multiple directions with respect to the longitudinal axis A of bridge 1226 even following insertion of the bridge 1226 into the slot 1230, when the arm actuator 1232 is in an unlocked configuration, which can allow the bar links 1204 to be placed at various angles with respect to the bar link 1204a.

When two pectus bars are used to correct a deformity, such as pectus excavatum, the bars (e.g., 1202) often do not perfectly align with each other (or are not parallel). The bars also may not be in the same plane and can be tilted or rotated with respect to each other. The features above allow the bar links 1204 to adapt to the misalignment between the two bars 1202. Also, these features (and optionally bending of the bridge 1226) can allow the bar links 1204 to be used with pectus bars installed in a crossing or X-configuration, or in other non-parallel configurations. When the bridge 1226 and bar link 1204a are in their desired positions, the arm actuator 1232 can be used to fix the orientation of the bridge 1226 with respect to the bar link 1204a.

This adjustability to tilt the bar links 1204 with respect to the bridge 1226 along with the bar links 1204 be adjustable along the pectus bars 1202, the bar links 1204 be adjustable along the bridge 1226, and the bar links 1204 be rotatable about the bridge 1226 allows for positioning or adjustability of the pectus bar assembly 1200 in every direction to allow for the pectus bar assembly 1200 to be installed in patient anatomy of a variety of shapes and sizes.

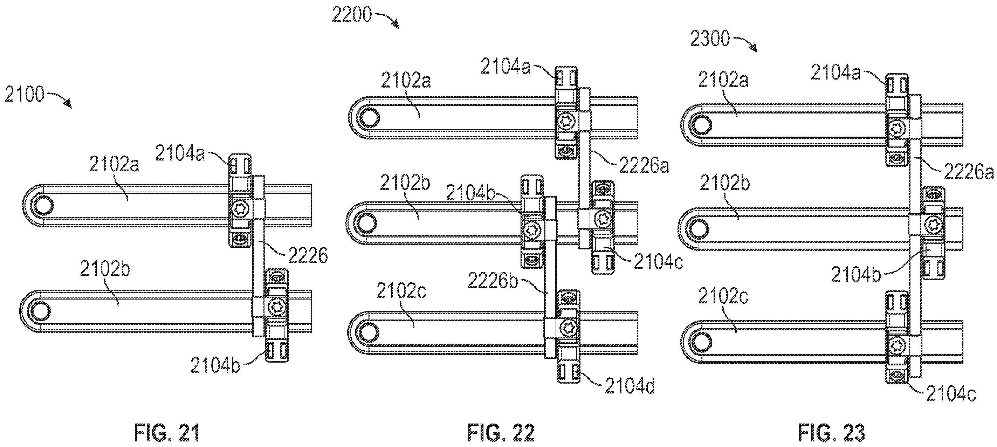

FIG. 21 illustrates an isometric view of a portion of a pectus bar assembly 2100. FIG. 22 illustrates an isometric view of a portion of a pectus bar assembly 2200. FIG. 23 illustrates an isometric view of a portion of a pectus bar assembly 2300. FIGS 21-23 are discussed together below. The pectus bar assembly 2100, the pectus bar assembly 2200, and the pectus bar assembly 2300, can be similar to the pectus bar assembly 1200 discussed above. FIGS. 21-23 show different ways that the assemblies can be used.

As shown in FIG. 21, the pectus bar assembly 2100 can include two pectus bars 2102a and 2102b, two stabilizers 2104a and 2104b, and a single bridge 2226. As shown in FIG. 22, the pectus bar assembly 2200 can include three pectus bars, 2102a and 2102b, four stabilizers 2104a-2104d, and two bridge 2226a and 2226b. In this configuration, the bar 2102b can receive two stabilizers, 2104b and 2104c to connect the bars 2102a and 2102c. As shown in FIGS. 23, the pectus bar assembly 2300 can include three pectus bars, 2102a and 2102b, but only three stabilizers 2104a-2104c, and one bridge 2226, allowing for less hardware to be used to interconnect three pectus bars. FIGS. 21-23 thereby show that the pectus bar assembly 2200 can be used in systems using one or more pectus bars, such as 1, 2, 3, 4, 5, or the like.

### Notes And Examples

The following, non-limiting examples, detail certain aspects of the present subject matter to solve the challenges and provide the benefits discussed herein, among others.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

In the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A pectus bar assembly comprising:
a first pectus bar (1202a) and a second pectus bar (1202b) engageable with a sternum;
a first bar link (1204a) connectable to the first pectus bar and translatable along the first pectus bar;
a second bar link (1204b) connectable to the second pectus bar and translatable along the second pectus bar; and
a bridge (1226) defining a longitudinal axis, the bridge connectable to the first bar link and the second bar link, the first bar link and the second bar link independently translatable along the bridge, and the first bar link and the second bar link independently rotatable about the longitudinal axis of the bridge.

2. The assembly of claim 1, the first bar link comprising:
a body (1228); and
a shelf (1224) connected to the body, the shelf configured to receive a first end portion of the first pectus bar therein.

3. The assembly of claim 2, the first bar link comprising:
an actuator (1234) extending through a bore (1246) of the body, the actuator operable between an unlocked position where the actuator is disengaged from the first pectus bar and a locked position where the actuator is engaged with a second end portion of the first pectus bar to cause engagement between the first end portion of the pectus bar and the shelf to secure the first bar link to the first pectus bar.

4. The assembly of claim 3, wherein the actuator is rotatable to cause the actuator to translate within the bore of the body.

5. The assembly of claim 4, wherein the actuator is captivated within the bore of the body.

6. The assembly of claim 4, wherein a distal portion of the actuator is shaped to avoid engagement with an anterior portion of the first pectus bar when the actuator is moved from the unlocked position to the locked position.

7. The assembly of claim 3, the first bar link comprising:
a head (1278) extending away from the body and away from the actuator, the head movable to deform the body to release the first bar link from the first pectus bar.

8. The assembly of claim 7, wherein the head includes a rib (1280) graspable to manipulate the first bar link with respect to the bridge and the first pectus bar.

9. The assembly of claim 8, the first bar link comprising:
an arm (1229) connected to the body, the arm and the body together defining a slot (1230) configured to receive the bridge at least partially therein; and
an arm actuator (1232) connected to the body and the arm and operable to move between a first position where the bridge is movable with respect to the body and a second position where the arm and the body engage the bridge to secure the first bar link to the bridge.

10. The assembly of claim 9, wherein the arm and the body together form a clamp.

11. The assembly of claim 10, wherein the arm is connected to the body by a living hinge (1231) configured to allow the arm to elastically deflect with respect to the body when the arm moves between the first position and the second position.

12. The assembly of claim 11, wherein the slot is a bore including an angled portion at an opening of the bore configured to allow the first bar link to tilt with respect to the longitudinal axis of the bridge when the bridge is positioned in the slot of the first bar link and the arm actuator is in the first position.

13. The assembly of claim 11, wherein the living hinge at least partially forms the slot.

14. The assembly of any of claims 1-13, wherein the bridge is a cylindrical rod.

## Patentansprüche

1. Pectus-Bar-Baugruppe, umfassend:
eine erste Pectus Bar (1202a) und eine zweiten Pectus Bar (1202b), die mit einem Brustbein in Eingriff bringbar sind;
ein erstes Stangenglied (1204a), das mit der ersten Pectus Bar verbindbar und entlang der ersten Pectus Bar verschiebbar ist;
ein zweites Stangenglied (1204b), das mit der zweiten Pectus Bar verbindbar und entlang der zweiten Pectus Bar verschiebbar ist; und
eine Brücke (1226), die eine Längsachse definiert, wobei die Brücke mit dem ersten Stangenglied und dem zweiten Stangenglied verbindbar ist, das erste Stangenglied und das zweite Stangenglied unabhängig entlang der Brücke verschiebbar sind und das erste Stangenglied und das zweite Stangenglied unabhängig um die Längsachse der Brücke drehbar sind.

2. Baugruppe nach Anspruch 1, wobei das erste Stangenglied Folgendes umfasst:
einen Körper (1228); und
eine Ablage (1224), die mit dem Körper verbunden ist, wobei die Ablage dazu konfiguriert ist, einen ersten Endabschnitt der ersten Pectus Bar darin aufzunehmen.

3. Baugruppe nach Anspruch 2, wobei das erste Stangenglied Folgendes umfasst:
einen Aktor (1234), der sich durch eine Bohrung (1246) des Körpers erstreckt, wobei der Aktor zwischen einer entriegelten Position, in welcher der Aktor von der ersten Pectus Bar gelöst ist, und einer verriegelten Position, in welcher der Aktor mit einem zweiten Endabschnitt der ersten Pectus Bar in Eingriff steht, betätigbar ist, um einen Eingriff zwischen dem ersten Endabschnitt der Pectus Bar und der Ablage zu bewirken, um das erste Stangenglied an der ersten Pectus Bar zu sichern.

4. Baugruppe nach Anspruch 3, wobei der Aktor drehbar ist, um zu bewirken, dass sich der Aktor innerhalb der Bohrung des Körpers verschiebt.

5. Baugruppe nach Anspruch 4, wobei der Aktor innerhalb der Bohrung des Körpers festgehalten ist.

6. Baugruppe nach Anspruch 4, wobei ein distaler Abschnitt des Aktors geformt ist, um einen Eingriff mit einem vorderen Abschnitt der ersten Pectus Bar zu vermeiden, wenn der Aktor aus der entriegelten Position in die verriegelte Position bewegt wird.

7. Baugruppe nach Anspruch 3, wobei das erste Stangenglied Folgendes umfasst:
einen Kopf (1278), der sich von dem Körper weg und von dem Aktor weg erstreckt, wobei der Kopf bewegbar ist, um den Körper zu verformen, um das erste Stangenglied von der ersten Pectus Bar zu lösen.

8. Baugruppe nach Anspruch 7, wobei der Kopf eine Rippe (1280) beinhaltet, die ergriffen werden kann, um das erste Stangenglied in Bezug auf die Brücke und die erste Pectus Bar zu manipulieren.

9. Baugruppe nach Anspruch 8, wobei das erste Stangenglied Folgendes umfasst:
einen Arm (1229), der mit dem Körper verbunden ist, wobei der Arm und der Körper zusammen einen Schlitz (1230) definieren, der dazu konfiguriert ist, die Brücke mindestens teilweise darin aufzunehmen; und
einen Armaktor (1232), der mit dem Körper und dem Arm verbunden und betätigbar ist, um sich zwischen einer ersten Position, in der die Brücke in Bezug auf den Körper bewegbar ist, und einer zweiten Position, in welcher der Arm und der Körper mit der Brücke in Eingriff stehen, zu bewegen, um das erste Stangenglied an der Brücke zu sichern.

10. Baugruppe nach Anspruch 9, wobei der Arm und der Körper zusammen eine Klemme bilden.

11. Baugruppe nach Anspruch 10, wobei der Arm durch ein Filmscharnier (1231) mit dem Körper verbunden ist, das dazu konfiguriert ist, es dem Arm zu ermöglichen, sich in Bezug auf den Körper elastisch zu biegen, wenn sich der Arm zwischen der ersten Position und der zweiten Position bewegt.

12. Baugruppe nach Anspruch 11, wobei der Schlitz eine Bohrung ist, die einen abgewinkelten Abschnitt an einer Öffnung der Bohrung beinhaltet, der dazu konfiguriert ist, es dem ersten Stangenglied zu ermöglichen, in Bezug auf die Längsachse der Brücke zu kippen, wenn die Brücke in dem Schlitz des ersten Stangenglieds positioniert ist und sich der Armaktor in der ersten Position befindet.

13. Baugruppe nach Anspruch 11, wobei das Filmscharnier mindestens teilweise den Schlitz bildet.

14. Baugruppe nach einem der Ansprüche 1-13, wobei die Brücke ein zylindrischer Stab ist.

## Revendications

1. Ensemble barre de pectus comprenant :
une première barre de pectus (1202a) et une seconde barre de pectus (1202b) pouvant se mettre en prise avec un sternum ;
une première liaison de barre (1204a) pouvant être reliée à la première barre de pectus et pouvant être translatée le long de la première barre de pectus ;
une seconde liaison de barre (1204b) pouvant être reliée à la seconde barre de pectus et pouvant être translatée le long de la seconde barre de pectus ; et
un pont (1226) définissant un axe longitudinal, le pont pouvant être relié à la première liaison de barre et à la seconde liaison de barre, la première liaison de barre et la seconde liaison de barre pouvant être translatées indépendamment le long du pont, et la première liaison de barre et la seconde liaison de barre pouvant tourner indépendamment autour de l'axe longitudinal du pont.

2. Ensemble selon la revendication 1, la première liaison de barre comprenant :
un corps (1228) ; et
un plateau (1224) relié au corps, le plateau étant conçu pour recevoir une première partie d'extrémité de la première barre de pectus.

3. Ensemble selon la revendication 2, la première liaison de barre comprenant :
un actionneur (1234) s'étendant à travers un alésage (1246) du corps, l'actionneur pouvant fonctionner entre une position déverrouillée dans laquelle l'actionneur est désaccouplé de la première barre de pectus et une position verrouillée dans laquelle l'actionneur est en prise avec une seconde partie d'extrémité de la première barre de pectus de manière à provoquer un mise en prise entre la première partie d'extrémité de la barre de pectus et le plateau afin de fixer la première liaison de barre à la première barre de pectus.

4. Ensemble selon la revendication 3, ledit actionneur pouvant tourner pour amener l'actionneur à se translater à l'intérieur de l'alésage du corps.

5. Ensemble selon la revendication 4, ledit actionneur étant capturé à l'intérieur de l'alésage du corps.

6. Ensemble selon la revendication 4, une partie distale de l'actionneur étant formée de manière à éviter une mise en prise avec une partie antérieure de la première barre de pectus lorsque l'actionneur est déplacé de la position déverrouillée à la position verrouillée.

7. Ensemble selon la revendication 3, la première liaison de barre comprenant :
une tête (1278) s'étendant en s'éloignant du corps et en s'éloignant de l'actionneur, la tête pouvant se déplacer de manière à déformer le corps afin de libérer la première liaison de barre de la première barre de pectus.

8. Ensemble selon la revendication 7, ladite tête comprenant une nervure (1280) pouvant être saisie pour manipuler la première liaison de barre par rapport au pont et à la première barre de pectus.

9. Ensemble selon la revendication 8, la première liaison de barre comprenant :
un bras (1229) relié au corps, le bras et le corps définissant ensemble une fente (1230) conçue pour recevoir le pont au moins partiellement en son sein ; et
un actionneur de bras (1232) relié au corps et au bras et pouvant fonctionner de manière à se déplacer entre une première position dans laquelle le pont est mobile par rapport au corps et une seconde position dans laquelle le bras et le corps se mettent en prise avec le pont de manière à fixer la première liaison de barre au pont.

10. Ensemble selon la revendication 9, ledit bras et ledit corps formant ensemble une pince.

11. Ensemble selon la revendication 10, ledit bras étant relié au corps par une charnière mobile (1231) conçue pour permettre au bras de fléchir de façon élastique par rapport au corps lorsque le bras se déplace entre la première position et la seconde position.

12. Ensemble selon la revendication 11, ladite fente étant un alésage comprenant une partie inclinée au niveau d'une ouverture de l'alésage conçue pour permettre à la première liaison de barre de s'incliner par rapport à l'axe longitudinal du pont lorsque le pont est positionné dans la fente de la première liaison de barre et que l'actionneur de bras se trouve dans la première position.

13. Ensemble selon la revendication 11, ladite charnière mobile formant au moins partiellement la fente.

14. Ensemble selon l'une quelconque des revendications 1 à 13, ledit pont étant une tige cylindrique.
